# EUROPEAN PATENT SPECIFICATION

(11) **EP 3 272 762 B1**
(45) Date of publication and mention of the grant of the patent: **10.06.2020**
(21) Application number: 16759047.0
(22) Date of filing: 04.03.2016
(51) Int. Cl.: B01J 20/22, B01J 20/30, C07H 15/203, C07F 1/00, C07F 1/04, C30B 29/58, C07H 15/20, C08B 37/00, C08K 5/151

(54) **POROUS POLYMER COMPOUND, SINGLE CRYSTALS, METHOD OF DETERMINING MOLECULAR STRUCTURE OF COMPOUND TO BE ANALYZED, AND METHOD OF DETERMINING ABSOLUTE CONFIGURATION OF CHIRAL COMPOUND**
PORÖSE POLYMERVERBINDUNG, EINKRISTALLE, VERFAHREN ZUR BESTIMMUNG DER MOLEKULAREN STRUKTUR EINER ZU ANALYSIERENDEN VERBINDUNG UND VERFAHREN ZUR BESTIMMUNG DER ABSOLUTEN KONFIGURATION EINER CHIRALEN VERBINDUNG
COMPOSÉ POLYMÈRE POREUX, MONOCRISTAUX, PROCÉDÉ DE DÉTERMINATION DE LA STRUCTURE MOLÉCULAIRE D'UN COMPOSÉ À ANALYSER ET PROCÉDÉ DE DÉTERMINATION DE LA CONFIGURATION ABSOLUE D'UN COMPOSÉ CHIRAL

(30) Priority: 04.03.2015 JP 2015042308
(43) Date of publication of application: 24.01.2018
(73) Proprietor: The University of Tokyo, Tokyo 113-8654 (JP)
(72) Inventor: FUJITA Makoto, Tokyo 113-8654 (JP); INOKUMA Yasuhide, Tokyo 113-8654 (JP)
(74) Representative: Schiener, Jens
(86) International application number: PCT/JP2016/056872
(87) International publication number: WO 2016/140355

(56) References cited:
- WO-A1-2010/104113
- WO-A1-2014/038220
- WO-A1-2014/038221
- JP-A- 2006 188 560
- US-A1- 2012 095 196
- KHAN SHADAB ALI ET AL: "Recent advances in the metallo-glycodendrimers and its potential applications", INORGANICA CHIMICA ACTA, vol. 409, 26 June 2013 (2013-06-26), pages 26-33, XP028763327, ISSN: 0020-1693, DOI: 10.1016/J.ICA.2013.06.017
- NOZOMI KAMIYA ET AL: "Saccharide-Coated M 12 L 24 Molecular Spheres That Form Aggregates by Multi-interaction with Proteins", JOURNAL OF THE AMERICAN CHEMICAL SOCIETY, vol. 129, no. 13, 1 April 2007 (2007-04-01), pages 3816-3817, XP055512938, US ISSN: 0002-7863, DOI: 10.1021/ja0693082
- ASANA TAKAHASHI ET AL.: '2 PA-193 Suiyosei M12L24 Kyujo Sakutai no Naimen Kannokika' DAI 92 ANNUAL MEETING OF THE CHEMICAL SOCIETY OF JAPAN IN SPRING(2012) KOEN YOKOSHU IV 09 March 2012, page 1661, XP009507372
- SOTA SATO ET AL.: '1 S 5-12 Tokubetsu Kikaku Koen Sakutai Nano Kukan no Seitai Bunshi o Tsukatta Jizai Shushoku' DAI 93 ANNUAL MEETING OF THE CHEMICAL SOCIETY OF JAPAN IN SPRING (2013) KOEN YOKOSHU I 08 March 2013, page 133, XP009507371

## Description

### TECHNICAL FIELD

The present invention relates to a porous polymer compound having a three dimensional skeleton comprising sugar derivatives and cations that interact at many points with hydroxyl groups and/or ether bonds of the sugar derivatives, and pores and/or voids that are partitioned and formed by the three dimensional skeleton; a single crystal of the porous polymer compound; a method of determining a molecular structure of a compound to be analyzed using the sample for crystal structure analysis; and a method of determining an absolute configuration of a chiral compound using the sample for crystal structure analysis.

### BACKGROUND ART

Porous polymer compounds are polymer compounds constituting a crystal having pores and/or voids therein. Such porous polymer compounds are known to have a property of selectively absorbing a specific compound in the pores and/or voids, and various uses have been suggested.

For example, Patent Literature 1 discloses a porous network complex comprising, as a structural unit, an M₆L₄ structure being constituted in a self-assembled manner by six transition metal ions (M) and four substantially planar tridentate organic ligands (L) and having an octahedral three-dimensional shape in which the transition metal ions (M) are positioned at six vertices thereof, wherein said M₆L₄ structures are consecutively arranged sharing the transition metal ions (M) positioned at the individual vertices thereof.

The literature discloses that the porous network complex can selectively absorb fullerene C₇₀ in its pores, and therefore fullerene C₇₀ can be separated by bringing the porous network complex into contact with a mixture of fullerenes containing fullerene C₆₀ and fullerene C₇₀.

Further, Non-patent Literature 1 discloses a method of preparing a sample for crystal structure analysis by using a porous single crystal of a polymer metal complex as a crystal sponge and having the polymer metal complex absorb flavonoid and the like into its pores, and determining its structure.

As mentioned above, porous polymer compounds are useful as tools in purification treatments and chemical analyses. Further, when a porous polymer compound is used for such purpose, an optimal porous polymer compound is preferably selected from among the many porous polymer compounds according to the size and properties of a target compound.

However, there are not so many porous polymer compounds reported to be usable for such purposes.

### CITATION LIST

### PATENT LITERATURE

Patent Literature 1: International Publication No. WO 2011/062260

### NON-PATENT LITERATURE

Non-Patent Literature 1: Monthly "Kagaku", Vol. 68, August 2013, pp. 35-40
US 2012/0095196 A1 discloses a polyhedral transition metal complex.

### SUMMARY OF INVENTION

### TECHNICAL PROBLEM

The present invention was developed in light of prior art mentioned above. An object of the invention is to provide a novel porous polymer compound, a single crystal of the porous polymer compound, a method of determining a molecular structure of a compound to be analyzed using the sample for crystal structure analysis, and a method of determining an absolute configuration of a chiral compound using the sample for crystal structure analysis.

### SOLUTION TO PROBLEM

The inventors conducted extensive studies of a novel porous polymer compound with a view to solving the problem mentioned above. As a result, the inventors found that a novel porous polymer compound can be obtained by using a compound containing sugar residues, and came to complete the present invention.

As such, the present invention provides porous polymer compounds of [1] to [3], single crystals of [4] and [5], a method of determining a molecular structure of a compound to be analyzed of [6], and a method of determining an absolute configuration of a chiral compound of [7], all mentioned below.

A porous polymer compound having a three-dimensional skeleton and pores and/or voids that are partitioned and formed by the three-dimensional skeleton in which the three-dimensional skeleton includes:
multiple sugar derivatives represented by formula (1) 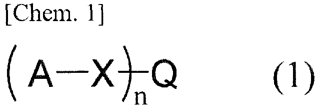
wherein A is a sugar residue having a carbon number of 5 to 30, and X is an oxygen atom or sulfur atom; Q is an organic group having a carbon number of 2 to 40 and a valence of n, forming a bond with A via X; n is an integer of 2 to 4; and a plurality of As and a plurality of Xs, respectively, may be the same or may be different; and multiple cations that interact with hydroxyl groups and/or ether bonds of the sugar derivatives, and
the three-dimensional skeleton is formed by each of the cations interacting with two or more sugar derivatives.

### ADVANTAGEOUS EFFECTS OF INVENTION

The present invention provides a novel porous polymer compound a single crystal of the porous polymer compound, a method of determining a molecular structure of a compound to be analyzed using the sample for crystal structure analysis, and a method of determining an absolute configuration of a chiral compound using the sample for crystal structure analysis.

### BRIEF DESCRIPTION OF DRAWINGS

FIG. 1 is a view illustrating the direction that a pore of a single crystal extends.
FIG. 2 is a view illustrating a porous polymer compound obtained in Example 1.
FIG. 3 is a view illustrating a porous polymer compound obtained in Example 2.
FIG. 4 is a view illustrating a porous polymer compound obtained in Example 3.
FIG. 5 is a view illustrating a porous polymer compound obtained in Example 4.
FIG. 6 is a view illustrating a porous polymer compound obtained in Example 5 after guest molecules are substituted.
FIG. 7 is a view illustrating a porous polymer compound obtained in Example 6.
FIG. 8 is a view illustrating a porous polymer compound obtained in Example 7 after guest molecules are substituted.

### DESCRIPTION OF EMBODIMENTS

The present invention is hereinafter described in detail in each of the following sections 1) novel porous polymer compound, 3) single crystal of porous polymer compound, method of determining molecular structure of compound to be analyzed, and method of determining absolute configuration of chiral compound.

Further, in the specification, pores and voids in a porous polymer compound may be referred to as "pores and/or voids in a porous polymer compound" or "pores and/or voids in a crystal".

### 1) Novel porous polymer compound

A porous polymer compound of the present invention is a porous polymer compound having a three-dimensional skeleton and pores and/or voids that are partitioned and formed by the three-dimensional skeleton. The three-dimensional skeleton comprises: multiple sugar derivatives represented by the formula (1) (hereinafter optionally referred to as "sugar derivatives (α)") and multiple cations that interact with hydroxyl groups and/or ether bonds of sugar derivatives (α), and the three-dimensional skeleton is formed by each of the cations interacting with two or more sugar derivatives.

The three-dimensional skeleton contained in the porous polymer compound of the present invention is a skeletal framework having a three-dimensional expanse inside the crystal, and comprises multiple sugar derivatives (α) and multiple cations that interact with hydroxyl groups and/or ether bonds of sugar derivatives (α), and is formed by each of the cations interacting with two or more sugar derivatives.

The number of sugar derivatives (α) and that of cations comprised in the porous polymer compound of the present invention are not particularly limited as long as the porous polymer compound is present as a crystal.

The "pores" and "voids" represent internal empty spaces inside the crystal. Internal empty spaces extending cylindrically are called "pores" and other internal empty spaces "voids".

The sugar derivatives (α) constituting the three-dimensional skeleton of the porous polymer compound of the present invention are compounds represented by formula (1) shown below.

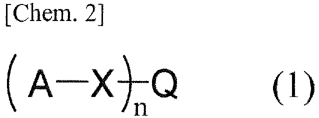

In formula (1), A is a sugar residue having a carbon number of 5 to 30, preferably 5 to 15. A sugar residue is a group made up of the remaining portion of a sugar molecule where any of the hydroxyl groups is removed. Since synthesis of sugar derivatives (α) is easy, a group made up of the remaining portion of a hemiacetal structure from which a hydroxyl group is removed is preferable.

Examples of the sugar residue of A may include sugar residues of monosaccharides and polysaccharides. Examples of the polysaccharides may include disaccharides, trisaccharides, tetrasaccharides and the like. From the perspective of availability and handleability, monosaccharides and disaccharides are preferable. Further, the above monosaccharides and polysaccharides may have optical isomers. The sugar residue may comprise only one type of any of the optical isomers, or it may comprise a mixture of optical isomers. In particular, the sugar residue comprising only one type of any of the optical isomers is preferable.

Examples of the sugar residue of the monosaccharides include an allose residue, altrose residue, galactose residue, glucose residue, gulose residue, idose residue, mannose residue, talose residue, arabinose residue, abequose residue, fucose residue, lyxose residue, mycarose residue, quinovose residue, rhamnose residue, ribose residue, paratose residue, xylose residue and the like.

Examples of the sugar residue of the disaccharides include a cellobiose residue, lactose residue, maltose residue, melibiose residue, maltitol residue and the like.

Examples of the sugar residue of an oligosaccharide of trisaccharides or higher include a nigerotriose residue, maltotriose residue, melezitose residue, maltotriulose residue, maltotriulose residue, kestose residue, raffinose residue, nystose residue, nigerotetraose residue, stachyose residue and the like.

Among these examples, sugar residues of disaccharides and monosaccharides are preferable, sugar residues of monosaccharides are more preferable, and a mannose residue or a glucose residue is even more preferable.

In formula (1), X is an oxygen atom or sulfur atom. Of the two, since synthesis of sugar derivatives (α) is easy, an oxygen atom is preferable.

In formula (1), Q is an organic group, having a carbon number of 2 to 40, preferably 2 to 20, and a valence of n, forming a bond with A via X.

An organic group of Q is preferably an organic group having an aromatic ring, since a more stable porous polymer compound is likely to be obtained. Further, when Q becomes larger, sugar residues of A will come to exist apart from each other, and therefore a porous polymer compound with relatively larger pores and voids is likely to be obtained.

Examples of the organic group of Q include, among others, divalent groups represented by formula (2) shown below, or trivalent groups represented by formula (3) shown below. Each "*" in formulae (2) and (3) shows a bond position with X.

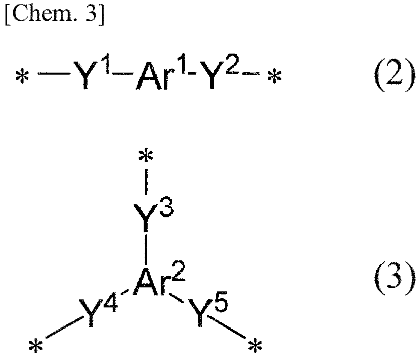

In formula (2), Ar¹ is a divalent aromatic group.

The number of carbon atoms constituting Ar¹ is ordinarily 3 to 22, preferably 3 to 13, and more preferably 3 to 6.

Examples of Ar¹ include divalent aromatic groups having a monocyclic structure, or divalent aromatic groups having a condensed ring structure formed by condensing two or more aromatic rings.

Examples of the divalent aromatic groups having a monocyclic structure include an o-phenylene group, m-phenylene group, p-phenylene group, pyridine-2, 5-diyl group, pyrazine-2, 5-diyl group and the like.

Examples of the divalent aromatic groups having a condensed ring structure formed by condensing two or more aromatic rings include groups represented by formulae (2a) to (2d) shown below. The two "*"s in each of formulae (2a) to (2d) show bond positions with Y¹ and Y².

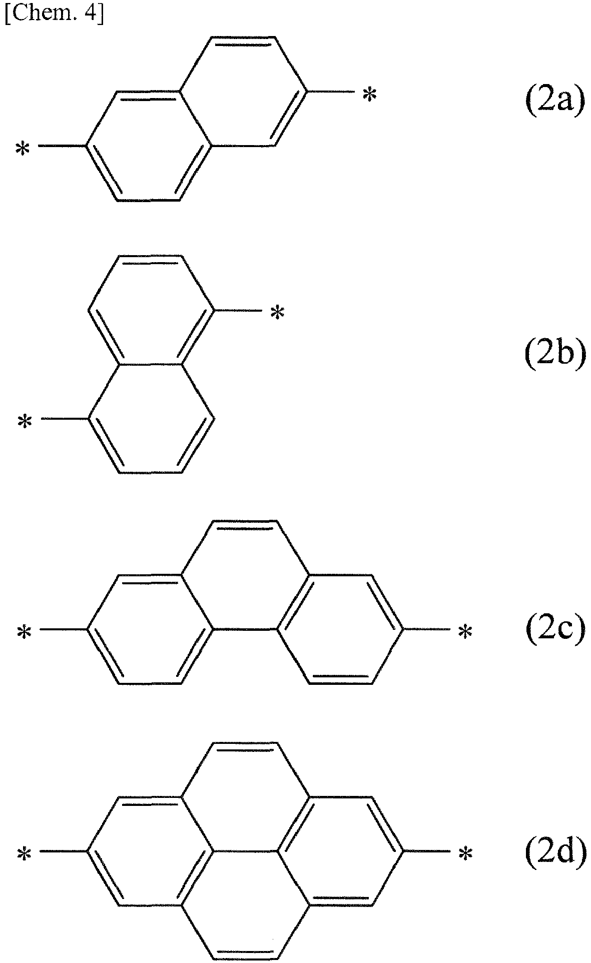

Ar¹ may be an aromatic group having a substituent at an optional position of the aromatic group. Examples of such a substituent include alkyl groups such as a methyl group, ethyl group, isopropyl group, n-propyl group, and t-butyl group; alkoxy groups such as a methoxy group, ethoxy group, n-propoxy group, and n-butoxy group; halogen atoms such as a fluorine atom, chlorine atom, and bromine atom; and the like.

A p-phenylene group is particularly preferable for Ar¹.

In formula (3), Ar² is a trivalent aromatic group.

The number of carbon atoms constituting Ar² is ordinarily 3 to 22, preferably 3 to 13, and more preferably 3 to 6.

Examples of Ar² include trivalent aromatic groups having a monocyclic structure comprising a six-membered aromatic ring.

Examples of the trivalent aromatic groups having a monocyclic structure comprising a six-membered aromatic ring include groups represented by formulae (3a) to (3d) shown below. Further, the three "*"s in each of formulae (3a) to (3d) show bond positions with Y³ to Y³.

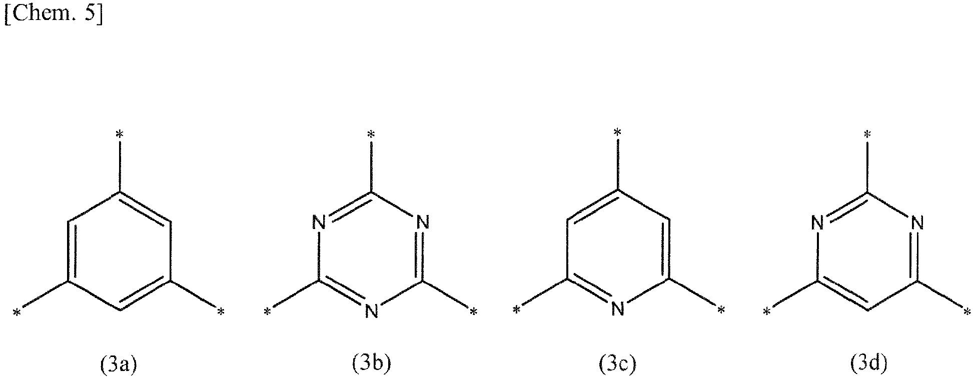

Ar² may be an aromatic group having a substituent at an optional position of the aromatic group. Examples of such a substituent include alkyl groups such as a methyl group, ethyl group, isopropyl group, n-propyl group, and t-butyl group; alkoxy groups such as a methoxy group, ethoxy group, n-propoxy group, and n-butoxy group; halogen atoms such as a fluorine atom, chlorine atom, and bromine atom; and the like.

A group represented by formula (3a) is preferable for Ar².

In formulae (2) and (3), Y¹ to Y⁵ each independently is a divalent organic group or a single bond.

The divalent organic group is preferably an organic group capable of constituting a π electron conjugated system with Ar¹ or Ar². As a result of the divalent organic groups represented by Y¹ to Y⁵ constituting a π electron conjugated system, planarity of sugar derivatives (α) improves, and a more solid three-dimensional skeleton is likely to be formed.

The number of carbon atoms constituting the divalent organic group is preferably 2 to 18, more preferably 2 to 12, and even more preferably 2 to 6.

Examples of the divalent organic group include divalent unsaturated aliphatic groups with a carbon number of 2 to 10, divalent organic groups having a monocyclic structure comprising a six-membered aromatic ring, divalent organic groups having a condensed ring structure formed by condensing two to four six-membered aromatic rings, amide groups [-C(=O)-NH-], ester groups [-C(=O)-O-], and combinations of two or more divalent organic groups thereof.

Examples of the divalent unsaturated aliphatic groups with a carbon number of 2 to 10 include a vinylene group, acetylene group (ethynylene group) and the like.

Examples of the divalent organic groups having a monocyclic structure comprising a six-membered aromatic ring, and the divalent organic groups having a condensed ring structure formed by condensing two to four six-membered aromatic rings include the same divalent aromatic groups as those of Ar¹.

Examples of the combinations of two or more divalent organic groups thereof include the followings.

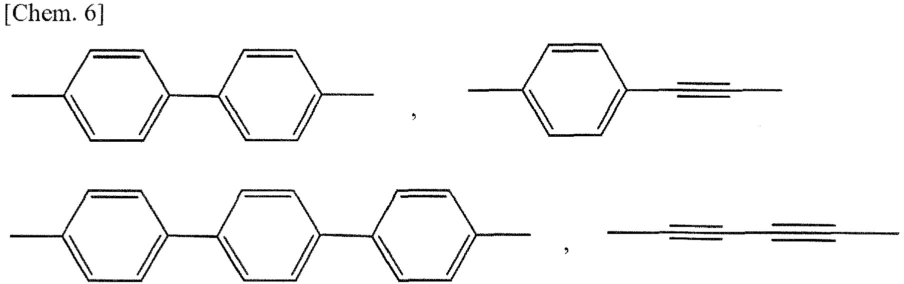

These aromatic rings may contain within the rings heteroatoms such as a nitrogen atom, oxygen atom, sulfur atom and the like.

Further, the divalent organic groups may have a substituent. Examples of such substituent include those disclosed above as substituents of Ar¹ and Ar².

By making appropriate selections for Ar¹, Ar², and Y¹ to Y⁵ in the groups represented by formula (2) and formula (3), sizes of pores and voids in the porous polymer compound can be adjusted. By applying this method, a porous polymer compound having pores and voids with sizes capable of accommodating molecules of a target compound can be effectively obtained.

In formula (1), n is an integer of 2 to 4, preferably 2 or 3. A plurality of As and a plurality of Xs, respectively, may be the same or may be different.

A sugar derivative (α) is preferably a compound represented by formulae shown below.

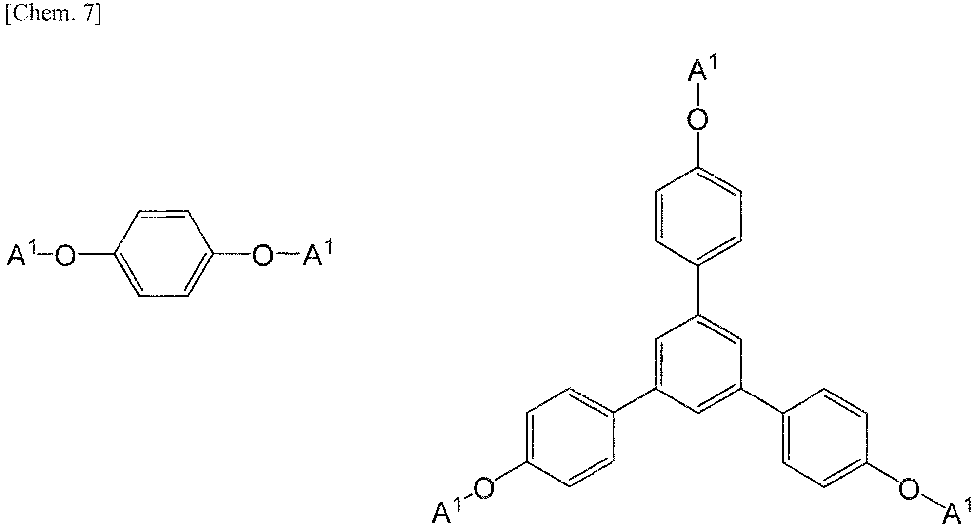

In the formulae, A¹ is a sugar residue of a sugar, preferably a sugar residue of a monosaccharide, and more preferably a mannose residue or a glucose residue.

A sugar derivative (α) may be a chiral compound with a known absolute configuration. As will be described later, an absolute configuration of a chiral compound (target compound) can be determined by using a single crystal of a porous polymer compound obtained by using a sugar derivative (α) which is a chiral compound with a known absolute configuration.

The sugar derivative (α) can be synthesized, for example, by reacting a compound containing a hydroxyl group corresponding to Q with an acetylated sugar in the presence of a catalyst.

Examples of cations include metal ions in group 1 of the periodic table such as a lithium ion, sodium ion, and potassium ion; metal ions in group 2 of the periodic table such as a magnesium ion, and calcium ion; metal ions in groups 8 to 12 of the periodic table such as an iron ion, cobalt ion, nickel ion, copper ion, zinc ion, silver ion, palladium ion, ruthenium ion, rhodium ion, and platinum ion; ammonium ions such as tetramethyl ammonium, and tetraethyl ammonium; phosphonium ions such as tetramethyl phosphonium, and tetraphenyl phosphonium; and the like.

Among the cations, metal ions in group 1 of the periodic table (alkali metal ions) are preferable.

The porous polymer compound of the present invention contains a large number of sugar derivatives (α) and a large number of cations. The cations interact with hydroxyl groups and/or ether bonds of the sugar derivatives (α), and form a three-dimensional skeleton of the porous polymer compound. One cation interacts with the surrounding two or more sugar derivatives (α). By way of such interaction, sugar derivatives (α) are fixed in a specific conformation, thus forming the three-dimensional skeleton.

At this point, the sugar derivatives (α) may be electrically neutral compounds, or may be deprotonated to become anions. This state, for example, is considered to be affected by Lewis acidity and the like of the cations, however, ordinarily the sugar derivatives (α) are electrically neutral compounds.

The sugar derivatives (α) in the interaction with the cations act as a hydrogen bond donor, hydrogen bond acceptor, Lewis base and the like.

For example, sodium ions can interact with oxygen atoms of hydroxyl groups and/or ether bonds of the sugar derivatives (α).

In the case where sugar derivatives (α) are electrically neutral compounds, the porous polymer compound of the present invention ordinarily contains anions. The anions are counter ions of the cations, thereby electrical balance can be maintained.

Examples of the anions include monovalent anions such as a hydroxyl ion (OH⁻), chloride ion (Cl⁻), bromide ion (Br⁻), iodide ion (I⁻), and thiocyanate ion (SCN⁻); divalent anions such as an oxide ion (O²⁻); polynuclear clusters such as Keggin type POM (polyoxometalate) [PMo₁₂O₄₀)³⁻, etc.]; and the like.

The anions can interact with hydrogen atoms and oxygen atoms of hydroxyl groups, and oxygen atoms of ether bonds of the sugar derivatives (α). For example, oxygen atoms and hydrogen atoms of hydroxide ions in sodium hydroxide can interact with hydrogen atoms and oxygen atoms, respectively, of hydroxyl groups of the sugar derivatives (α).

Further, the porous polymer compound of the present invention may contain electrically neutral compounds besides the cations and anions. Examples of the electrically neutral compounds include coordinating compounds such as water, ammonia, monoalkylamine, dialkylamine, trialkylamine, and ethylenediamine.

In the case where cations, anions or electrically neutral compounds have large volumes, a porous polymer compound with relatively large pores and voids is likely to be obtained.

The porous polymer compound of the present invention may contain guest molecules in its pores and/or voids. Examples of such guest molecules include, for example, solvent molecules used in synthesis. Further, the guest molecules are interchangeable with other molecules, and, for example, when a porous polymer compound is immersed in a solvent with high affinity to the guest molecules, the guest molecules are released from the pores and/or voids.

A method of synthesizing a porous polymer compound is not particularly limited, and a well-known method can be employed.

For example, a brochure issued by Sigma-Aldrich in September, 2012 (Basics of Materials Science, Vol. 7, Basics of Porous Coordination Polymers (PCP)/Metal Organic Frameworks (MOF)) discloses a solution method comprising mixing a solution containing a multidentate ligand and the like, and a solution containing metal ions and the like; a hydrothermal method comprising adding a solvent, multidentate ligand, metal ions and the like into a pressure resistant container, sealing the pressure resistant container, and heating the pressure resistant container to above the boiling point of the solvent to conduct a hydrothermal reaction; a microwave method comprising adding a solvent, multidentate ligand, metal ions and the like into a container, and irradiating the solution with a microwave; an ultrasonic wave method comprising adding a solvent, multidentate ligand, metal ions and the like into a container, and irradiating the solution with ultrasonic wave; a solid phase synthetic method comprising mechanically mixing a multidentate ligand, metal ions and the like without using a solvent; and the like. A porous polymer compound can be obtained using one of these methods.

Among these methods, the solution method is preferably used, since it does not require any special device and the like.

Examples of the solution method include, for example, a method comprising adding a solution of a raw material compound (hereinafter optionally and simply referred to as a "raw material compound") that produces cations into a solution of sugar derivatives (α), and allowing the solution to stand as it is for a few hours to a few days at 0 to 70°C.

The ratio of the amount of the sugar derivatives (α) and the raw material compound in molar ratio of "sugar derivatives (α): raw material compound" is ordinarily 1:1 to 1:1000, preferably 1:1 to 1:50.

Concentration of the solution of the sugar derivatives (α) is not particularly limited, however, it is ordinarily 0.0001 to 10 mol/L, preferably 0.001 to 0.1 mol/L.

Concentration of the solution of the raw material compound is not particularly limited, however, it is ordinarily 0.0001 to 10 mol/L, preferably 0.001 to 0.1 mol/L.

The raw material compound is not particularly limited as long as it produces cations when dissolved. For example, a compound represented by formula MXₘ is exemplified. Here, M is a cation such as a metal ion, X is an anion, and m is the valence of M.

Examples of M mentioned above are not particularly limited, however, the examples include, for example, metal ions in group 1 of the periodic table such as Li⁺, Na⁺, K⁺, Rb⁺, and Cs⁺; metal ions in group 2 of the periodic table such as Be²⁺, Mg²⁺, Ca²⁺, Sr²⁺, and Ba²⁺; metal ions in groups 8 to 12 of the periodic table such as Fe²⁺, Fe³⁺, Co²⁺, Co³⁺, Ni²⁺, Cu²⁺, Zn²⁺, Ag⁺, Pd²⁺, Ru²⁺, Ru³⁺, Rh²⁺, Rh³⁺, Pt²⁺; ammonium ions such as tetramethyl ammonium, and tetraethyl ammonium; phosphonium ions such as tetramethyl phosphonium, and tetraphenyl phosphonium; and the like.

Specific examples of X mentioned above are not particularly limited, however, the examples include, for example, OH⁻, F⁻, Cl⁻, Br⁻, I⁻, SCN⁻, NO₃⁻, ClO₄⁻, BF₄⁻, SbF₄⁻, PF₆⁻, AsF₆⁻, CH₃CO₂⁻ and the like.

Examples of the reaction solvents used, i.e., a solvent of a solution of a sugar derivative (α) and a solvent of a solution of a raw material compound, include aromatic hydrocarbons such as benzene, toluene, xylene, chlorobenzene, 1,2-dichlorobenzene, and nitrobenzene; aliphatic hydrocarbons such as n-pentane, n-hexane, and n-heptane; alicyclic hydrocarbons such as cyclopentane, cyclohexane, and cycloheptane; nitriles such as acetonitrile, and benzonitrile; sulfoxides such as dimethyl sulfoxide (DMSO); amides such as N,N-dimethylformamide, and n-methylpyrrolidon; ethers such as diethyl ether, tetrahydrofuran, 1,2-dimethoxyethane, and 1,4-dioxane; alcohols such as methanol, ethanol, and isopropyl alcohol; ketones such as acetone, methyl ethyl ketone, and cyclohexanone; cellosolves such as ethylcellosolve; halogenated hydrocarbons such as dichloromethane, chloroform, carbon tetrachloride, and 1,2-dichloroethane; esters such as methyl acetate, ethyl acetate, ethyl lactate , and ethyl propionate; water; and the like. These solvents may be used independently or in a combination of two or more. Among these solvents, water, ethanol, methanol, diethyl ether, dimethyl sulfoxide, and tetrahydrofuran are preferable.

As will be mentioned below, the porous polymer compound of the present invention may be utilized as an absorber used in separating a compound to be separated, or as a crystal sponge used in preparing a sample for crystal structure analysis.

### 2) Method of separating a compound to be separated

Such a method is characterized in that a porous polymer compound is contacted with a mixture containing a compound to be separated, thus allowing molecules of the compound to be separated to be absorbed into the pores and/or voids in the porous polymer compound.

An example of the method, where a porous polymer compound is contacted with a mixture containing a compound to be separated, thus allowing molecules of the compound to be separated to be absorbed into the pores and/or voids of the porous polymer compound, include a method similar to that of preparing a sample for crystal structure analysis to be described below.

In other words, other than the aspects where it is not necessary to use a single crystal, or it is not necessary for a compound to be separated to be absorbed with regularity into the pores and/or voids in a porous polymer compound, in the method of separating a compound and in the method of preparing a sample for crystal structure analysis, molecules of a target compound are absorbed in the pores and/or voids in the porous polymer compound by a similar operation.

Therefore, contact conditions between the porous polymer compound and the mixture in the method of separating a compound may be determined on the basis of the contact conditions in the method of preparing a sample for crystal structure analysis to be described below. Further, in the of separating a compound, molecules of the compound to be separated may be randomly absorbed in the pores and/or voids in the porous polymer compound by using a high-concentration solution of the compound to be separated or by decreasing the duration of contact.

In the method of separating a compound, after the molecules of the compound to be separated are absorbed in the pores and/or voids in the porous polymer compound, the absorbed molecules of the compound to be separated are released into a solvent having affinity with the compound to be separated by immersing the porous polymer compound into the solvent. Therefore, for example, the compound to be separated of high-purity can be obtained by releasing the molecules of the compound to be separated into the solvent, then filtering out the porous polymer compound and vaporizing the solvent from the resulting filtrate.

3) Single crystal of porous polymer compound, method of preparing sample for crystal structure analysis, method of determining molecular structure of compound to be analyzed, and method of determining absolute configuration of chiral compound

### Single crystal of porous polymer compound

A single crystal of the present invention consists of the porous polymer compound mentioned above.

The single crystal of the porous polymer compound is a single crystal having the three-dimensional skeleton mentioned above, and pores and/or voids that are partitioned and formed by the three-dimensional skeleton, and that are three-dimensionally and regularly arranged.

The "pores and/or voids ... that are three-dimensionally and regularly arranged" mean regularly arranged pores or voids without randomness to the extent the pores or voids can be confirmed by crystal structure analysis.

The single crystal of the present invention can be synthesized by using a method similar to a conventional method of preparing a single crystal of a porous polymer compound. For example, a single crystal can be obtained 1) by using a solvent of a solution of a sugar derivative (α) and a solvent of a solution of a raw material compound that have no compatibility with each other, i.e. they separate into two-layers, 2) by having the solutions stand still in layers, or 3) by performing synthesis using a lower concentration condition, in the method of synthesizing a porous polymer compound previously mentioned.

The size of a pore is correlated to the diameter of an inscribed circle of the pore (hereinafter optionally referred to as an "inscribed circle of the pore") in a plane (hereinafter optionally referred to as a "parallel plane") parallel to a crystal plane which is closest to perpendicular to the direction in which the pore extends. The larger the inscribed circle is, the larger the pore size would be. The smaller the inscribed circle is, the smaller the pore size would be.

The "direction in which the pore extends" can be determined by the following method.

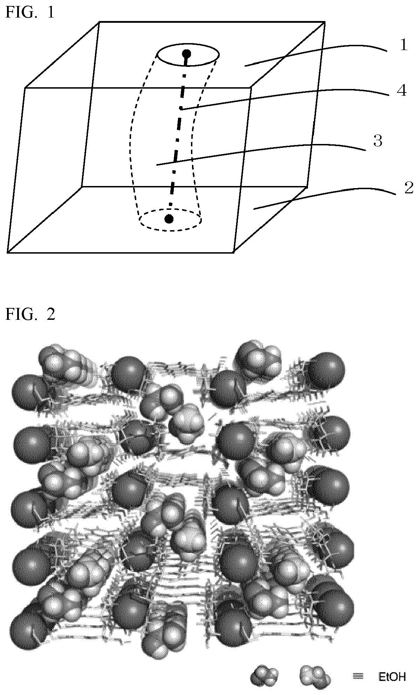

Namely, a crystal plane X, for example, A plane, B plane, C plane or a diagonal plane of each plane, of an adequate direction crossing a target pore is first selected. Then, a cross-sectional diagram of the pore with crystal plane X being a cutting plane is drawn by indicating an atom present on crystal plane X and constituting the three-dimensional skeleton using van der Waals radius. Similarly, a cross-sectional diagram of the pore with crystal plane Y, which is one unit cell moved from the crystal plane X, being a cutting plane is drawn. Then, a straight line (an alternate long and short dash line) in a three-dimensional view drawing is drawn between the centers of a cross-sectional shape of the pore in each crystal plane (see Fig. 1). The direction of the resulting straight line is the direction in which the pore extends.

Further, the "diameter of an inscribed circle of the pore" can be found by the following method.

Namely, by a method similar to the method mentioned above, a cross-sectional diagram of the pore with the parallel plane being a cutting plane is first drawn. Then, an inscribed circle of the pore is drawn in the cross-sectional diagram, and after measuring its diameter, the actual diameter of the inscribed circle of the pore can be found by converting the obtained measurement to the actual scale.

Further, the diameter of an inscribed circle at the narrowest portion and the diameter of an inscribed circle at the widest portion can be found by measuring the diameter of an inscribed circle of the pore in each parallel plane while gradually translating the parallel plane for one unit cell.

The diameter of an inscribed circle of the pore in the single crystal is preferably 2 to 30 Å, and more preferably 3 to 10 Å.

Further, in the case where the shape of the pore is very different from a perfect circle, it is desirable to anticipate the inclusion action of a single crystal from the minor diameter and major diameter of an inscribed ellipse of the pore in the parallel plane.

The major diameter of the inscribed ellipse of the pore in the single crystal is preferably 2 to 30 Å, and more preferably 3 to 10 Å. Further, the minor diameter of the inscribed ellipse of the pore in the single crystal is preferably 2 to 30 Å, and more preferably 3 to 10 Å.

The pore volume in the single crystal can be found by a procedure disclosed in Paper (A): Acta Crystallographica Section A, Vol. 46, pp. 194-201, 1990. Namely, it can be calculated using "Volume of single crystal × Porosity in unit cell" on the basis of Solvent Accessible Void (void volume in unit lattice) calculated by a calculation program (PLATON SQUEEZE PROGRAM).

Pore volume in the single crystal (volume of all pores in a grain of a single crystal) is preferably 1 × 10⁻⁷ to 0.1 mm3, and more preferably 1 × 10⁻⁵ to 1 × 10⁻³ mm³.

Further, in a case the single crystal has voids, the size of the voids can also be found by the procedure disclosed in Paper (A) mentioned above, similarly to the procedure for the case of pore volume.

The single crystal preferably has a shape of a cube or a rectangular solid. Its side length is preferably 10 to 1,000 µm, and more preferably 60 to 200 µm. A quality sample for crystal structure analysis is likely to be obtained by using a single crystal of such shape and size.

The single crystal may only comprise a three-dimensional skeleton (so-called host molecules), or it may have a three-dimensional skeleton and interchangeable molecules (so-called guest molecules) in the pores and/or voids.

The single crystal is preferably capable of determining a molecular structure with a resolution of at least 1.5 Å, when an MoK alpha radiation (wavelength: 0.71 Å) generated at tube voltage of 24 kV and tube current of 50 mA is irradiated and a diffraction X-ray is detected with a CCD detector. A quality sample for crystal structure analysis is likely to be obtained by using a single crystal having such properties.

The single crystal may be one obtained by using a sugar derivative (α) which is a chiral compound with a known absolute configuration. As will be described later, the absolute configuration of a chiral compound (target compound) can be determined by using such single crystal.

### Method of preparing sample for crystal structure analysis

The method of preparing a sample for crystal structure analysis is characterized in that the single crystal is contacted with a compound to be analyzed, thus allowing molecules of the compound to be analyzed to be regularly arranged in the pores and/or voids in a porous polymer compound.

The size of the compound to be analyzed is not particularly limited as long as the compound to be analyzed is of the size that can be accommodated in the pores and/or voids in the single crystal. Molecular weight of the compound to be analyzed is ordinarily 20 to 3,000, and preferably 100 to 2,000.

It is also preferable to understand to a certain extent the size of molecules of the compound to be analyzed in advance by nuclear magnetic resonance spectrometry, mass spectrometry, element analysis and the like, and adequately select and use a single crystal having pores and voids of appropriate size.

A method of bringing the single crystal into contact with the compound to be analyzed is not particularly limited. Examples of the method, for example, include 1) a method where a solution of the compound to be analyzed is prepared and the single crystal is brought into contact with the solution, and 2) a method where the single crystal is directly brought into contact with the compound to be analyzed in the case the compound to be analyzed is liquid or gas. Molecules of the compound to be analyzed can be absorbed into the pores and/or voids in the porous polymer compound by employing these methods. Among these methods, the method where a solution of the compound to be analyzed is prepared and the single crystal is brought into contact with the solution is preferable, since a better quality sample for crystal structure analysis is likely to be obtained.

Further, in the case of using a solution containing the compound to be analyzed, or in the case where the compound to be analyzed is liquid, contact operation can be performed, among others, by a method of immersing the single crystal into a solution containing the compound to be analyzed and the like, or by a method where the single crystal is stuffed into a capillary, and then, a solution containing the compound to be analyzed or the like is passed through the inside of the capillary.

A solvent of a solution containing the compound to be analyzed is adequately selected from among those that do not dissolve a single crystal to be used, and that dissolve the compound to be analyzed.

Specific examples of the solvent to be used include aromatic hydrocarbons such as benzene, toluene, xylene, chlorobenzene, 1,2-dichlorobenzene, and nitrobenzene; aliphatic hydrocarbons such as n-butane, n-pentane, n-hexane, and n-heptane; alicyclic hydrocarbons such as cyclopentane, cyclohexane, and cycloheptane; nitriles such as acetonitrile, and benzonitrile; sulfoxides such as dimethyl sulfoxide (DMSO); amides such as N,N-dimethylformamide, and n-methylpyrrolidon; ethers such as diethyl ether, tetrahydrofuran, 1,2-dimethoxyethane, and 1,4-dioxane; alcohols such as methanol, ethanol, and isopropyl alcohol; ketones such as acetone, methyl ethyl ketone, and cyclohexanone; cellosolves such as ethylcellosolve; halogenated hydrocarbons such as dichloromethane, chloroform, carbon tetrachloride, and 1,2-dichloroethane; esters such as methyl acetate, ethyl acetate, ethyl lactate, and ethyl propionate; water; and the like. These solvents may be used independently or in a combination of two or more.

The amount of the compound to be analyzed used is not particularly limited, however ordinarily it is 1 ng to 100 g, and preferably 1 µg to 1 g.

The duration of contact is not particularly limited, however ordinarily it is 1 minute to 50 days, preferably 6 hours to 7 days.

The temperature at the time of contact is not particularly limited, however ordinarily it is -20 to 150°C, preferably 0 to 50°C.

The sample for crystal structure analysis obtained by the method of the present invention comprises molecules of the compound to be analyzed being regularly arranged in the pores and/or voids in the single crystal.

The "molecules of the compound to be analyzed being regularly arranged" mean that molecules of the compound to be analyzed are regularly accommodated in the pores and voids in the single crystal without randomness to the extent the structure can be determined by crystal structure analysis.

The method of regularly accommodating molecules of the compound to be analyzed in the pores and voids in the single crystal is not particularly limited, however, for example, the sample for crystal structure analysis comprising molecules of the compound to be analyzed being regularly arranged is more likely to be obtained by using a solution of relatively low concentration of the compound to be analyzed, or by sufficiently increasing the duration of contact.

The sample for crystal structure analysis is preferably capable of determining a molecular structure with a resolution of at least 1.5 Å, when an MoK alpha radiation (wavelength: 0.71 Å) generated at tube voltage of 24 kV and tube current of 50 mA is irradiated and a diffraction X-ray is detected with a CCD detector.

Molecules of the compound to be analyzed do not need to be absorbed in all pores and voids in the single crystal as long as the sample for crystal structure analysis is capable of determining the molecular structure of the compound to be analyzed. For example, a solvent used in a solution containing the compound to be analyzed may be absorbed in a portion of pores and voids in the single crystal.

In the sample for crystal structure analysis, occupancy of molecules of the compound to be analyzed is preferably 10% or higher.

Occupancy is a value obtained by crystal structure analysis, and it represents the amount of guest molecules actually present in a single crystal when the amount of guest molecules (molecules of a compound to be analyzed) at an ideal inclusion state is 100%.

Further, in the case where the compound to be analyzed is a chiral compound, a sample for crystal structure analysis for determining the absolute configuration of the chiral compound can be produced by using a single crystal obtained using a sugar derivative (α) which is a chiral compound with a known absolute configuration.

### Method of determining molecular structure of compound to be analyzed

The method of determining the molecular structure of a compound to be analyzed of the present invention is characterized by performing crystal structure analysis of the compound to be analyzed using a sample for crystal structure analysis obtained by the method mentioned above.

In the method of the present invention, either method of X-ray diffraction or neutron diffraction can be employed.

When determining the molecular structure of the compound to be analyzed by the method of the present invention, a method similar to a conventional method can be employed, except for mounting the sample for crystal structure analysis obtained by the method mentioned above instead of a conventional single crystal.

### Method of determining absolute configuration of chiral compound

The method of determining the absolute configuration of a chiral compound of the present invention involves performing similar operations to those of the method of determining a molecular structure mentioned above. However, in the former method, unlike in the latter method, the compound to be analyzed is a chiral compound, and the single crystal used is obtained by using sugar derivatives (α) which are chiral compounds with a known absolute configuration.

In general, according to X-ray crystal structure analysis method, relative positional relations between atoms and distances between atoms and the like can be understood, however it is difficult to determine the absolute configuration of a chiral compound.

On the other hand, according to the determining method of the present invention, the absolute configuration of sugar derivatives (α) is known, and therefore the absolute configuration of a target chiral compound can be easily determined on the basis of relative positional relations with sugar derivatives (α).

In other words, similar to a conventionally performed method of determining an absolute configuration using an asymmetric auxiliary group, i.e., a method of determining an absolute configuration of a target chiral compound, where an asymmetric auxiliary group with a known absolute configuration is introduced into a molecule of a chiral compound, and relative positional relations with the asymmetric auxiliary group are studied, the absolute configuration of a chiral compound can be determined.

According to the method of the present invention, the absolute configuration of a chiral compound can be efficiently determined.

In particular, the method of the present invention can be used even in the case where the amount of a chiral compound is very small. Therefore, absolute configurations of very small amounts of impurities or various metabolites contained in medicines, agrochemicals, and their raw materials can be efficiently determined.

### EXAMPLES

The present invention is hereinafter described in more detail with examples. Further, the present invention shall not be limited in any means by the following examples. Hereinafter, a "sugar derivative" may optionally be referred to as a "ligand".

Devices
(1) Single crystal X-ray structure analysis
   Performed with Bruker APEX II CCD Diffractometer [Radiation source: Mo-K alpha radiation (wavelength 0.71 Å), Output: 50 mA, 24 kV]
(2) Element analysis
   Performed with MT-6 from YANACO
(3) NMR measurements
   Performed with Bruker DRX-500
(4) ESI-mass measurements
   Performed with maXis from Bruker

### Production Example 1: Synthesis of ligand 1

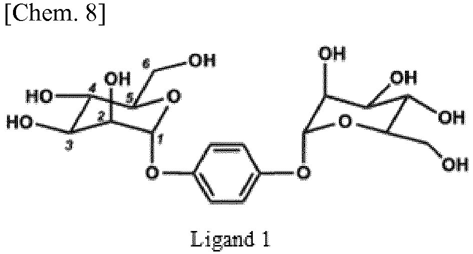

Penta-O-acetyl-D-mannose (1.95 g, 5.00 mmol) and hydroquinone (275 mg, 2.50 mmol) were dissolved in 20 ml of dichloromethane in a 50 ml eggplant flask, and to the resulting solution was added boron trifluoride-diethylether complex (0.63 mL), and the resulting mixture was stirred for 24 hours at 20°C. The reaction solution was washed with 5% sodium hydrogen carbonate solution (40 mL) and water (20mL), dried with anhydrous sodium sulfate, and the solvent was distilled with a rotary evaporator.

Crude product was purified with a recycle type size exclusion chromatography, and deca-O-acetylated product of ligand 1 was obtained [yield (amount) 1.0 g, yield (rate) 52%].

Then, deca-O-acetylated product of ligand 1 (200 mg, 0.26 mmol) and sodium methoxide (1.5 mg, 0.026 mmol) were dissolved in 10 ml of methanol, and the solution was stirred for 1 hour at 20°C. The resulting solution was neutralized by being passed through an ion-exchange resin, Dowex 50X2-200, and then, insoluble matter was filtered out, volatile components in the filtrate were distilled with a rotary evaporator, and ligand 1 was obtained [yield (amount) 110 mg, yield (rate) 98%].

Physical properties of ligand 1 are shown below.

¹H-NMR (500MHz, MeOD): δ (ppm) 7.05 (s, 4H, Ar-H), 5.37 (d, J = 1.5 Hz, 2H, H₁), 3.99 (dd, J₁₋₂ = 1.5 Hz, J₂₋₃ = 3.5 Hz, 2H, H₂), 3.88 (dd, J₃₋₂ = 3.5 Hz, J₃₋₄ = 9. 5 Hz, 2H, H₃), 3.79-3.70 (m, 6H, H₄, H₆), 3.62 (ddd, J₅₋₆ = 2. 5 Hz, J₅₋₆ = 4. 5 Hz, J₅₋₄ = 7 Hz, 2H, H₅)
¹³C-NMR (125 MHz, MeOD): δ (ppm) 153.3, 119.0, 100.9, 75.3, 72.4, 72.1, 68.4, 62.7
IR (cm⁻¹) 3327 (br), 1505 (s), 1434 (m), 1210 (s), 1117 (s), 1049 (m), 1005 (s), 975 (s), 883 (m), 824 (m), 776 (m), 721 (m), 676 (m)
ESI mass m/z calcd for C₁₈H₂₆O₁₂: 457. 1316 [M+Na]⁺; found: 457. 1319
Elemental analysis (%): calcd for C₁₈H₂₆O₁₂•H₂O: C47.79, H6.24, N0.00; found: C47.89, H6.29, N0.00.

### Production Example 2: Synthesis of ligand 2

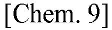

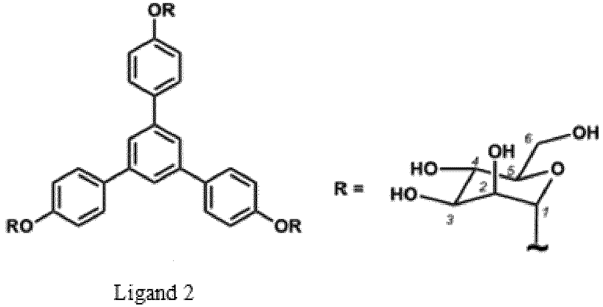

Penta-O-acetyl-D-mannose (3.96 g, 10.15 mmol) and 1,3,5-tri(4-hydroxyphenyl) benzene (1.2 g, 3.36 mmol) were dissolved in 120 ml of dichloromethane in a 200 ml eggplant flask, and boron trifluoride-diethylether complex (16.8 mL) and 510 mg of triethylamine were added to the resulting solution, and the resulting mixture was stirred for 38 hours at 60°C. The reaction solution was washed with 5% sodium hydrogen carbonate solution (40 mL) and water (20mL), dried with anhydrous sodium sulfate, and the solvent was distilled with a rotary evaporator.

Crude product was purified with a recycle type size exclusion chromatography, and pentadeca-O-acetylated product of ligand 2 was obtained [yield (amount) 3.0 g, yield (rate) 67%].

Then, pentadeca-O-acetylated product of ligand 2 (800 mg, 0.595 mmol) and sodium methoxide (6.7 mg, 0.12 mmol) were dissolved in 100 ml of methanol, and the solution was stirred for 3 hours at 20°C. The resulting solution was neutralized by being passed through an ion-exchange resin, Dowex 50, and then, insoluble matter was filtered out, volatile components in the filtrate were distilled with a rotary evaporator, and ligand 2 was obtained [yield (amount) 490 mg, yield (rate) 98%].

Physical properties of ligand 2 are shown below.

¹H-NMR (500MHz, MeOD): δ (ppm) 7.66 (s, 3H, Ar-H), 7.64 (d, J = 8. 5 Hz, 6H, Ar-H), 7.23 (d, J = 8.5 Hz, 6H, Ar-H), 5.52 (dd, J₁₋₂ = 1.5 Hz, 3H, H₁), 4.04 (dd, J₂₋₁ = 1.5 Hz, J₂₋₃ = 3.5 Hz, 3H, H₂), 3.94 (dd, J₃₋₂ = 3.5 Hz, J₃₋₄ = 9 Hz, 3H, H₃) 3.81-3.72 (m, 9H, H₄ and H₆), 3.66-3.63 (m, 3H, H₅)
¹³C-NMR (125 MHz, CDCl₃): δ (ppm) 157.6, 143.1, 136.6, 129.4, 124.8, 118.2, 75.4, 72.4, 72.0, 68.4, 62.7
IR (cm⁻¹) 3332 (br), 2906 (m), 1607 (m), 1507 (s), 1444 (m), 1224 (s), 1181 (m), 1097 (m), 1000 (s), 986 (s), 883 (m), 825 (m), 782 (m), 670 (m)
ESI mass m/z calcd for C₄₂H₄₈O₁₈: 863.27 [M+Na]⁺; found: 863.70.

### Production Example 3: Synthesis of ligand 3

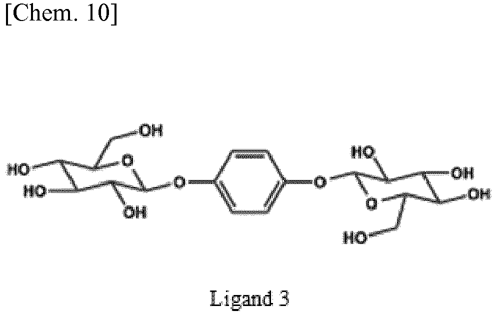

Ligand 3 was synthesized according to a method disclosed in the following literature.
Xu, Z.; Lee, S.; Lobkovsky, E. B.; Kiang, Y. -H., Journal of the American Chemical Society, 2002, Vol. 124, pp. 121-135

### Example 1

A solution of water 1 mL/ethanol 4 mL of ligand 1 (8.7 mg, 0.02 mmol) was put into a test tube with an inner diameter of 1 cm and a height of 10 cm, and 1 mL of diethyl ether was gently poured onto the solution in the test tube. Further, sodium hydroxide aqueous solution (4.8 mg/1 mL) was gently poured onto the diethyl ether layer in the test tube. The test tube was capped and was allowed to stand for 1 week at 20°C. Crystals precipitated on the wall of the test tube. The crystals were filtered [yield (amount) 14 mg, yield (rate) 85%] and various measurements were made.

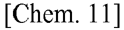

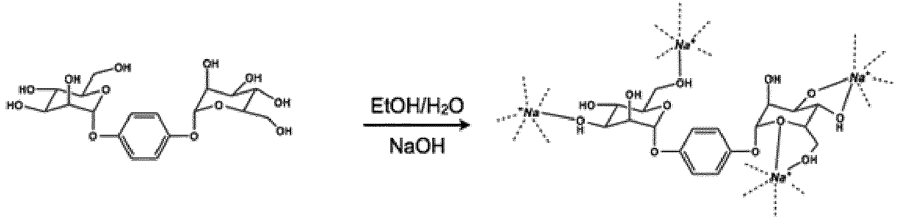

Physical properties are shown below.

IR (cm⁻¹): 3390 (br), 1507 (s), 1355 (m), 1208 (s), 1115 (m), 1020 (m), 1067 (m), 1047 (m), 1015 (s), 950 (s), 923 (m), 880 (m), 823 (m), 779 (m), 720 (m), 683 (m)
Elemental analysis: calcd for [(C₁₈H₂₆O₁₂) (NaOH) (EtOH)_{0.5}(H₂O)₃]ₙ: C41.38, H6.58, N0. 00; found: C41.52, H6.40, N0. 00

Results of crystal structure analysis are shown in Table 1 and Fig. 2. Further, Fig. 2 to Fig. 8 which is described later are separately submitted in color figures according to the prescribed written.

**Table 1**

| Crystal System | Monoclinic |
|---|---|
| Space Groups | P21 |
| a (Å) | 6.6333 (8) |
| b (Å) | 24.632 (3) |
| c (Å) | 14.7364 (17) |
| α (° ) | 90 |
| β (° ) | 101.7370 (10) |
| γ (° ) | 90 |
| Z | 2 |
| R1 | 7.12 |

### Example 2

A solution of water 1 mL/ethanol 4 mL of ligand 1 (8.7 mg, 0.02 mmol) was put into a test tube with an inner diameter of 1 cm and a height of 10 cm, and 1 mL of diethyl ether was gently poured onto the solution in the test tube. Further, potassium hydroxide aqueous solution (6.7 mg/1 mL) was gently poured onto the diethyl ether layer in the test tube. The test tube was capped and was allowed to stand for 1 week at 20°C. Crystals precipitated on the wall of the test tube. The crystals were filtered [yield (rate) 84%] and various measurements were made.

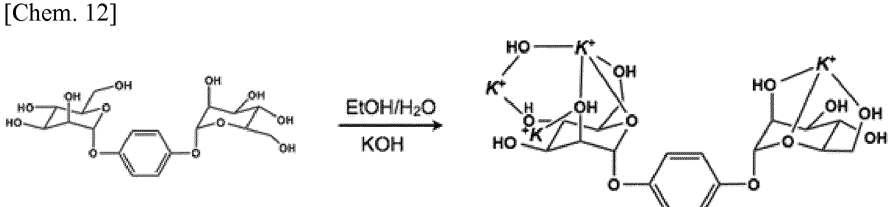

Physical properties are shown below.

IR (cm⁻¹): 3212 (br), 2921 (m), 1507 (s), 1225 (s), 1116 (s), 1020 (s), 976 (s), 822 (s), 669 (m), 625 (m)
Elemental analysis: calcd for [(C₁₈H₂₆O₁₂) (KOH) (EtOH) (H₂O)₃]ₙ: C41.38, H6.58, N0. 00; found: C41.52, H6.40, N0. 00.

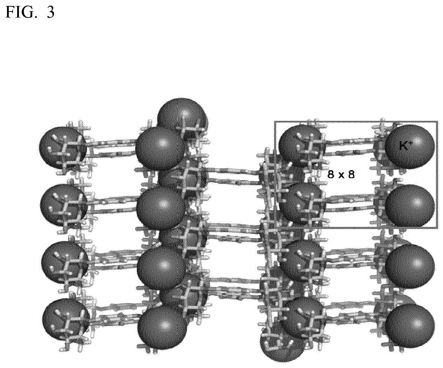

Results of crystal structure analysis are shown in Table 2 and Fig. 3.

**Table 2**

| Crystal System | Orthorhombic |
|---|---|
| Space Groups | P212121 |
| a (Å) | 7.4001 (18) |
| b (Å) | 12.375 (3) |
| c (Å) | 27.537 (7) |
| α (° ) | 90 |
| β (° ) | 90 |
| γ (° ) | 90 |
| Z | 4 |
| R1 | 10.88 |

### Example 3

Ligand 2 (16.8 mg, 0.02 mmol) and rubidium hydroxide (3.1 mg, 0.03 mmol) were dissolved in a mixed solvent of water 1 mL/ethanol 1 mL/dimethylsulfoxide 1 mL in a test tube with an inner diameter of 1 cm and a height of 10 cm. 1mL of diethyl ether was gently poured onto the resulting solution, and further, 3 ml of methanol was gently poured onto the diethyl ether layer in the test tube. The test tube was capped and was allowed to stand for 1 week at 20°C. Crystals precipitated on the wall of the test tube. The crystals were filtered and various measurements were made.

Physical properties are shown below.

IR (cm⁻¹): 3344 (br), 2900 (m), 1607 (s), 1508 (s), 1396 (m), 1227 (s), 1181 (m), 1112 (m), 1067 (m), 1045 (m), 1009 (s), 976 (s), 885 (m), 826 (s), 784 (m), 684 (m)

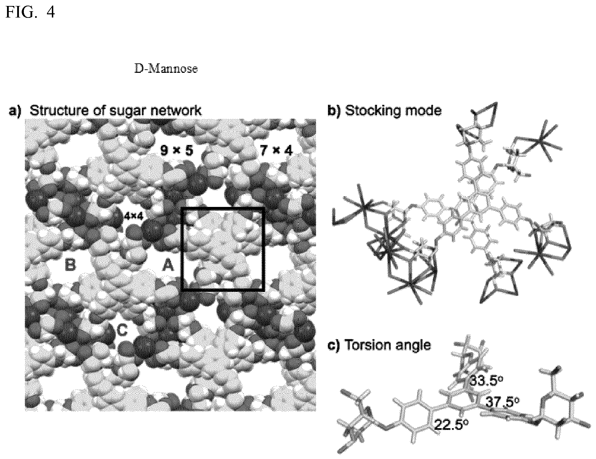

Results of crystal structure analysis are shown in Table 3 and Fig. 4.

**Table 3**

| Crystal System | Monoclinic |
|---|---|
| Space Groups | C2 |
| a (Å) | 72.928 (15) |
| b (Å) | 7.6386 (16) |
| c (Å) | 21.238 (5) |
| α (° ) | 90 |
| β (° ) | 95.004 (3) |
| γ (° ) | 90 |
| Z | 4 |
| R1 | 11.57 |

### Example 4

Ligand 3 (21.9 mg, 0.05 mmol) and sodium hydroxide (80 mg, 2 mmol) were dissolved in 1 mL of water in a test tube with an inner diameter of 1 cm and a height of 10 cm. 0.5 mL of diethyl ether was gently poured onto the resulting solution, and further, 4 mL of ethanol was gently poured onto the diethyl ether layer in the test tube. The test tube was capped and was allowed to stand for 1 week at 20°C. Crystals precipitated on the wall of the test tube. The crystals were filtered [yield (amount) 27 mg, yield (rate) 93%] and various measurements were made.

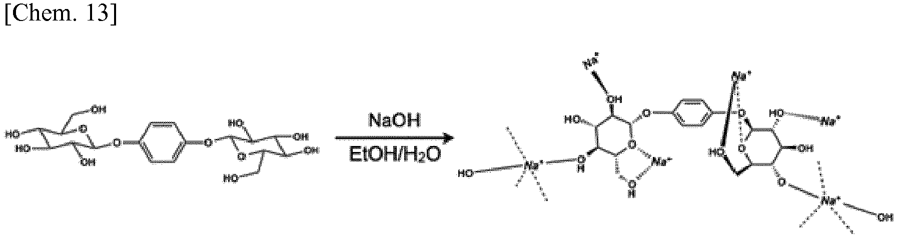

Physical properties are shown below.

IR (cm⁻¹): 3410 (br), 1611 (m), 1506 (s), 1379 (m), 1348 (m), 1309 (m), 1222 (m), 1156 (m), 1112 (m), 1092 (m), 1062 (s), 1007 (s), 909 (m), 826 (m), 794 (m), 675 (m), 637 (m), 623 (m), 576 (m)
Elemental analysis: calcd for [(C₁₈H₂₆O₁₂)(NaOH)₂(H₂O)₃]ₙ: C38.03, H6.03, N0. 00; found: C38.02, H6.03, N0. 00.

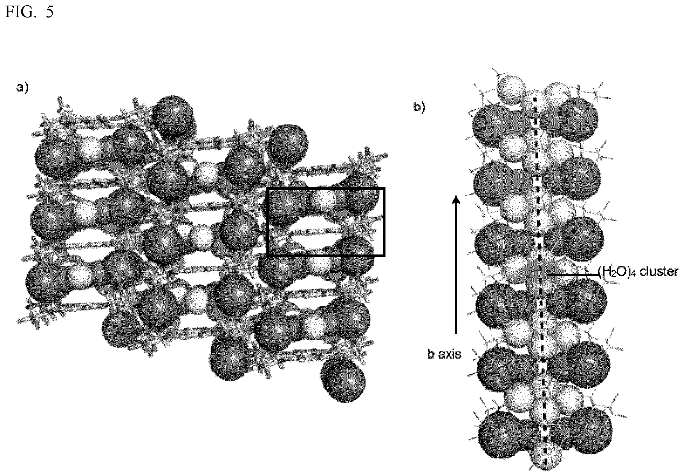

Results of crystal structure analysis are shown in Table 4 and Fig. 5.

**Table 4**

| Crystal System | Monoclinic |
|---|---|
| Space Groups | C2 |
| a (Å) | 26.526 (9) |
| b (Å) | 6.490 (2) |
| c (Å) | 7.068 (2) |
| α (° ) | 90 |
| β (° ) | 97.575 (4). |
| γ (° ) | 90 |
| Z | 4 |
| R1 | 3.62 |

### Example 5

10 mL of chloroform was put into a test tube, and 70 mg of crystals of the porous polymer compound obtained in Example 1 were immersed in the chloroform.
The test tube was capped and was allowed to stand for 10 days at 50°C. Meanwhile, an operation of removing 95% (volume ratio) of the supernatant solution with a dropper, and adding pure chloroform in the same volume as that of the removed solution was repeated every day.

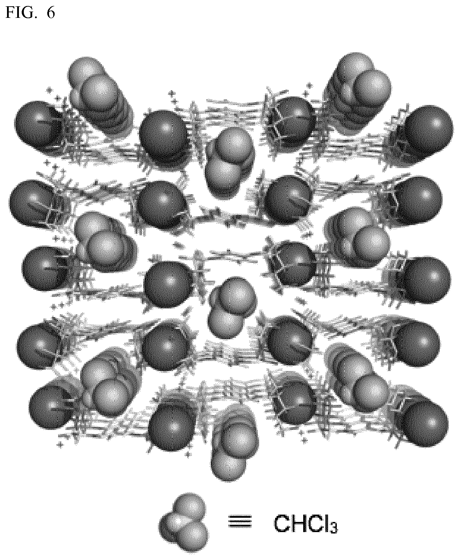

After filtering the crystals, elemental analysis and crystal structure analysis were performed. As a result, it was found that molecules in the pores were substituted with chloroform molecules. Results of crystal structure analysis are shown in Table 5 and Fig. 6.

Elemental analysis: calculated for [(C₁₈H₂₆O₁₂)₂(NaOH)₂(CHCl₃)(H₂O)₃]ₙ: C40.92, H5.29, N0. 00; found: C41.05, H5.23, N0.00

**Table 5**

| Crystal System | Monoclinic |
|---|---|
| Space Groups | P21 |
| a (Å) | 6.5829 (10) |
| b (Å) | 24.974 (4) |
| c (Å) | 14.585 (2) |
| α (° ) | 90 |
| β (° ) | 102.544 (2) |
| γ (° ) | 90 |
| Z | 2 |
| R1 | 5.51 |

### Example 6

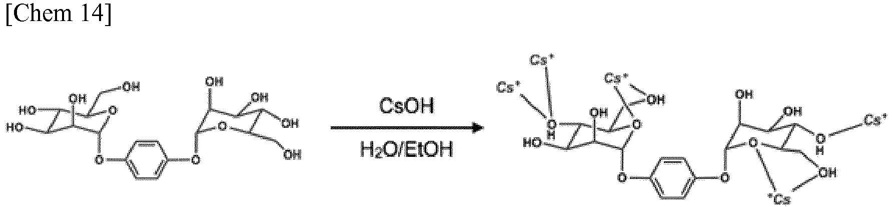

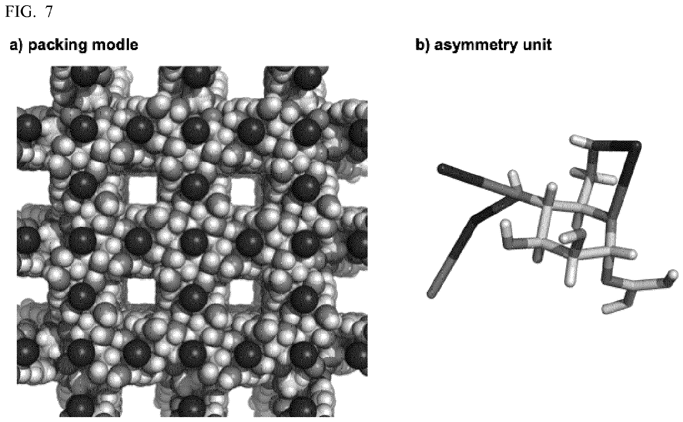

Ligand 1 (8.68 mg, 0.02 mmol) was dissolved in 1 mL of water and 4 mL of ethanol in a test tube with an inner diameter of 1 cm and a height of 10 cm. 1 mL of diethyl ether was gently poured onto the resulting solution, and further, 1.0 mL of cesium hydroxide aqueous solution of 120 mM was gently poured onto the diethyl ether layer in the test tube. The test tube was capped and was allowed to stand for 1 week at 20°C. Crystals precipitated on the wall of the test tube. The crystals were taken out and single crystal X-ray structure analysis was performed. Results are shown in Table 6 and Fig. 7.

**Table 6**

| Crystal System | Monoclinic |
|---|---|
| Space Groups | P422 |
| a (Å) | 14.332 (2) |
| b (Å) | 14.332 (2) |
| c (Å) | 12.1909 (19) |
| α (° ) | 90 |
| β (° ) | 90 |
| γ (° ) | 90 |
| Z | 1 |
| R1% | 10.16 |

### Example 7

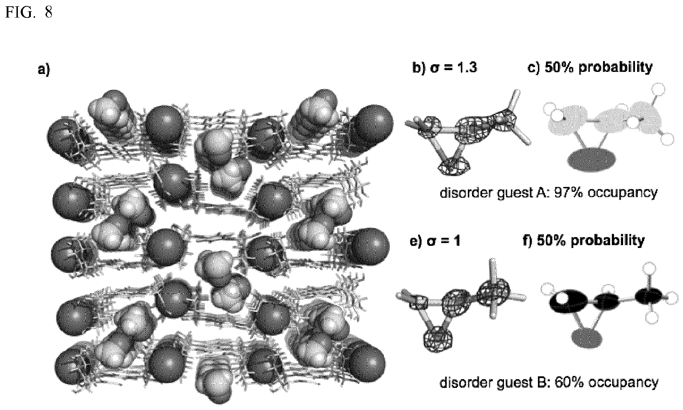

0.5 mL of (R)-propylene oxide was put in a test tube, and about 5 mg of crystals of the porous polymer compound obtained in Example 1 were immersed in the (R)-propylene oxide. The test tube was capped and was allowed to stand for 5 days at 50°C. Meanwhile, an operation of removing 95% (volume ratio) of the supernatant solution with a dropper, and adding pure (R)-propylene oxide in the same volume as that of the removed solution was repeated about every 30 hours. The crystals were taken out and single crystal X-ray structure analysis was performed. Results of the analysis are shown in Table 7 and Fig. 8.

**Table 7**

| Crystal System | Monoclinic |
|---|---|
| Space Groups | P21 |
| a (Å) | 6.632 (2) |
| b (Å) | 24.679 (9) |
| c (Å) | 14.699 (5) |
| α (° ) | 90 |
| β (° ) | 102.402 (4) |
| γ (° ) | 90 |
| Z | 4 |
| R1 | 6.97 |

Further, the resulting crystals including (R)-propylene oxide was dissolved in heavy water/methanol-d₄ (at volume ratio of 1:9), and the mole ratio of ligand 1 to (R)-propylene oxide was found to be 1/0.8.

Further, in the porous polymer compound obtained in Example 1, the absolute configuration of ligand 1 is known similarly to that of D-mannose, and therefore, by comparing the relative configuration of propylene oxide in the crystal structure in Example 7 with that of ligand 1, it can be confirmed that the absorbed propylene oxide is an (R)-form.

### REFERENCE SIGNS LIST

- 1:: Crystal plane X
- 2:: Crystal plane Y
- 3:: Pore
- 4:: Direction of extension of pore

## Claims

1. A porous polymer compound having a three-dimensional skeleton and pores and/or voids that are partitioned and formed by the three-dimensional skeleton, wherein the three-dimensional skeleton comprises
multiple sugar derivatives represented by formula (1) 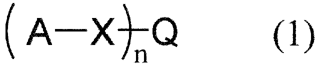 wherein
A is a sugar residue having a carbon number of 5 to 30, and
X is an oxygen atom or sulfur atom;
Q is an organic group, having a carbon number of 2 to 40 and a valence of n, forming a bond with A via X;
n is an integer of 2 to 4; and
a plurality of As and a plurality of Xs may be identical or may be different; and multiple cations that interact with hydroxyl groups and/or ether bonds of the sugar derivatives, and
the three-dimensional skeleton is formed by each of the cations interacting with two or more sugar derivatives.

2. The porous polymer compound according to claim 1, wherein Q is a group having an aromatic ring.

3. The porous polymer compound according to claim 1, wherein the cations are alkali metal ions.

4. A single crystal consisting of the porous polymer compound according to any one of claims 1.

5. The single crystal according to claim 4, wherein the sugar derivative represented by the formula (1) is a chiral compound with a known absolute configuration.

6. A method of determining a molecular structure of a compound to be analyzed comprising preparing a sample for crystal structure analysis, wherein the single crystal according to claim 4 or 5 is contacted with a compound to be analyzed, thus allowing molecules of the compound to be analyzed to be regularly arranged in the pores and/or voids of the porous polymer compound, wherein then a crystal structure analysis is conducted on the prepared sample.

7. A method of determining an absolute configuration of a chiral compound comprising preparing a sample for crystal structure analysis for determining an absolute configuration of a chiral compound, wherein the single crystal according to claim 5 is contacted with a chiral compound whose absolute configuration is to be determined, thus allowing molecules of the chiral compound whose absolute configuration is to be determined to be regularly arranged in the pores and/or voids of the porous polymer compound, wherein then the absolute configuration of the chiral compound is determined by a crystal structure analysis method on the prepared sample.

## Patentansprüche

1. Poröse Polymerverbindung mit einem dreidimensionalen Gerüst und Poren und/oder Hohlräumen, die aufgeteilt sind und durch das dreidimensionale Gerüst gebildet werden, wobei das dreidimensionale Gerüst folgendes umfasst: 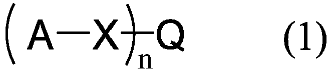 mehrere Zuckerderivate der Formel (1)
wobei
A für einen Zuckerrest steht, der eine Kohlenstoffzahl von 5 bis 30 aufweist und
X für ein Sauerstoffatom oder ein Schwefelatom steht;
Q für eine organische Gruppe mit einer Kohlenstoffzahl von 2 bis 40 steht und einer Valenz von n, die eine Bindung mit A über X bildet;
n für eine ganze Zahl von 2 bis 4 steht und
eine Vierzahl von A und eine Vielzahl von X gleich oder verschieden sein können;
und mehrere Kationen, die mit Hydroxylgruppen und/oder Etherbindungen der Zuckerderivate wechselwirken, und wobei das dreidimensionale Gerüst von jedem der Kationen gebildet wird, die mit zwei oder mehr Zuckerderivaten wechselwirken.

2. Poröse Polymerverbindung nach Anspruch 1, wobei Q für eine Gruppe mit einem aromatischen Ring steht.

3. Poröse Polymerverbindung nach Anspruch 1, wobei die Kationen Alkalimetallionen sind.

4. Einkristall, bestehend aus der porösem Polymerverbindung nach Anspruch 1.

5. Einkristall nach Anspruch 4, wobei das Zuckerderivat der Formel (1) eine chirale Verbindung mit einer bekannten absoluten Konfiguration ist.

6. Verfahren zur Bestimmung der molekularen Struktur einer zu analysierenden Verbindung, umfassend das Herstellen einer Probe für eine Kristallstrukturanalyse, wobei der Einkristall nach Anspruch 4 oder 5 mit der zu analysierenden Verbindung in Kontakt gebracht wird, so dass ermöglicht wird, dass die Moleküle der zu analysierenden Verbindung regelmäßig in den Poren und/oder Hohlräumen der porösen Polymerverbindung angeordnet werden, wobei dann eine Kristallstrukturanalyse bei der hergestellten Probe durchgeführt wird.

7. Verfahren zur Bestimmung der absoluten Konfiguration einer chiralen Verbindung, umfassend das Herstellen einer Probe für die Kristallstrukturanalyse, um die absolute Konfiguration einer chiralen Verbindung zu bestimmen, wobei der Einkristall nach Anspruch 5 mit der chiralen Verbindung, deren absolute Konfiguration bestimmt werden soll, in Kontakt gebracht wird, so dass ermöglicht wird, dass die Moleküle der chiralen Verbindung, deren absolute Konfiguration bestimmt werden soll, regelmäßig in den Poren und/oder Hohlräumen der porösen Polymerverbindung angeordnet werden, wobei dann die absolute Konfiguration der chiralen Verbindung durch ein Verfahren der Kristallstrukturanalyse bei der hergestellten Probe bestimmt wird.

## Revendications

1. Composé polymère poreux ayant une charpente tridimensionnelle et des pores et/ou vides qui sont répartis et formés par la charpente tridimensionnelle, dans lequel la charpente tridimensionnelle comprend
de multiples dérivés de sucre représentés par la formule (1) 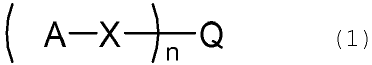 dans laquelle
A est un résidu de sucre ayant de 5 à 30 carbones, et
X est un atome d'oxygène ou un atome de soufre ;
Q est un groupe organique ayant de 2 à 40 carbones et une valence de n, formant une liaison avec A via X ;
n est un entier de 2 à 4 ; et
plusieurs A et plusieurs X peuvent être identiques ou différents ; et
de multiples cations qui interagissent avec des groupes hydroxyle et/ou des liaisons éther des dérivés de sucre, et
la charpente tridimensionnelle est formée par chacun des cations interagissant avec deux ou plus de deux dérivés de sucre.

2. Composé polymère poreux selon la revendication 1, dans lequel Q est un groupe ayant un cycle aromatique.

3. Composé polymère poreux selon la revendication 1, dans lequel les cations sont des ions de métal alcalin.

4. Monocristal consistant en le composé polymère poreux de la revendication 1.

5. Monocristal selon la revendication 4, dans lequel le dérivé de sucre représenté par la formule (1) est un composé chiral ayant une configuration absolue connue.

6. Procédé pour déterminer la structure moléculaire d'un composé devant être analysé, comprenant la préparation d'un échantillon pour une analyse de structure cristalline, dans lequel le monocristal selon la revendication 4 ou 5 est mis en contact avec un composé devant être analysé, ce qui permet ainsi à des molécules du composé à analyser d'être disposées régulièrement dans les pores et/ou vides du composé polymère poreux, dans lequel ensuite une analyse de structure cristalline est effectuée sur l'échantillon préparé.

7. Procédé pour déterminer la configuration absolue d'un composé chiral, comprenant la préparation d'un échantillon pour une analyse de structure cristalline afin que soit déterminée la configuration absolue d'un composé chiral, dans lequel le monocristal selon la revendication 5 est mis en contact avec un composé chiral dont la configuration absolue doit être déterminée, ce qui permet ainsi à des molécules du composé chiral dont la configuration absolue doit être déterminée d'être disposées régulièrement dans les pores et/ou vides du composé polymère poreux, dans lequel ensuite la configuration absolue du composé chiral est déterminée par un procédé d'analyse de structure cristalline sur l'échantillon préparé.
